# EUROPÄISCHE PATENTANMELDUNG

(11) **EP 3 854 334 A1**
(43) Veröffentlichungstag der Anmeldung: **28.07.2021**
(21) Anmeldenummer: 20153421.1
(22) Anmeldetag: 23.01.2020
(51) Int. Cl.: A61B 18/02, F17C 5/06

(54) **GERÄT ZUR SPEISUNG EINES MEDIZINISCHEN INSTRUMENTS MIT EINEM KÄLTEMITTEL**

(71) Anmelder: Erbe Elektromedizin GmbH, 72072 Tübingen (DE)
(72) Erfinder: MAYER, Volker,, 72072 Tuebingen (DE); STRAUB, Frank,, 72072 Tübingen (DE); FRITZ, Martin,, 72070 Tuebingen (DE); WANDEL, Waldemar,, 72127 Kusterdingen (DE)
(74) Vertreter: Rüger Abel Patentanwälte PartGmbB

(57) **Zusammenfassung**

Ein Gerät (10) zur Speisung kryochirurgischer Instrumente mit einem Kältemittel, insbesondere CO₂, das vorzugsweise in Flaschen bereitgestellt wird, weist eine Pumpe (17) auf, um das der Flasche entnommene CO₂ in einem Puffergefäß (19) mit einem gewünschten Betriebsdruck bereitzustellen, wobei der Druck in der Gasflasche kleiner sein kann als der gewünschte Betriebsdruck. Das Gerät (10) umfasst eine Temperierungseinrichtung (27), die dazu eingerichtet ist, das Kältemittel auf eine gewünschte Temperatur, insbesondere eine Temperatur zu bringen, die höher ist als die Temperatur in der Gasflasche oder einem sonstigen Vorratsgefäß (15). Damit wird eine sichere Versorgung eines kryochirurgischen Instruments mit Kältemittel des gewünschten Drucks und einer ausreichenden Temperatur sichergestellt. Insbesondere werden unerwünschte Tröpfchenbildungen in dem Instrument oder zuführenden Leitungen unterbunden.

## Beschreibung

Die Erfindung betrifft ein Gerät und ein Verfahren zur Speisung eines medizinischen Instruments mit einem Kältemittel.

Zur Versorgung medizinischer Instrumente mit einem Kältemittel schlägt die US 2002/0068929 A1 vor, aus einer Druckflasche entnommenes Gas, mittels einer Kolbenpumpe zu verdichten. Diese Verdichtung hat insbesondere dann Sinn, wenn der Gasdruck in der Vorratsflasche allmählich abnimmt, sodass er für den Betrieb eines Kryoinstruments oder sonstigen Verbrauchers nicht mehr ausreicht. Der Gasinhalt der Flasche wird deswegen nur unvollständig genutzt. Mit der Kolbenpumpe kann jedoch auch dann Gas mit dem gewünschten Druck zur Verfügung gestellt werden, wenn der Druck in der Vorratsflasche unter dem geforderten Druck abgefallen ist.

Die Druckerhöhung kann jedoch bei Gasen, die als gesättigter Dampf vorliegen, zu Fehlfunktionen im Instrument oder auch an anderen Aggregaten, wie zum Beispiel Druckreglern, Ventilen oder dergleichen führen.

Es ist Aufgabe der Erfindung, ein Gerät und ein Verfahren zur Speisung eines medizinischen Instruments mit einem Kältemittel bereitzustellen, bei dem der ordnungsgemäße Betrieb des Instruments auch dann sichergestellt ist, wenn der Druck eines in einem Vorratsgefäß gehaltenen Kältemittels geringer ist, als zum Betrieb des Instruments erforderlich.

Diese Aufgabe wird mit einem Gerät nach Anspruch 1 und einem Verfahren nach Anspruch 14 gelöst:

Das erfindungsgemäße Gerät weist ein Vorratsgefäß für einen Kältemittelvorrat oder einen Anschluss für ein solches Vorratsgefäß auf, in dem das Kältemittel in komprimierter Form vorliegt. Weiter umfasst das erfindungsgemäße Gerät ein Puffergefäß, in dem ein weiterer Kältemittelvorrat bereitgehalten werden kann. Auch in dem Puffergefäß steht das Kältemittel unter Druck, wobei dieser Druck größer ist oder sein kann, als der Druck in dem Vorratsgefäß.

Weiter ist eine Temperiereinrichtung vorgesehen, die mindestens mit dem Puffergefäß verbunden ist, um dessen Temperatur zu beeinflussen. Auf diese Weise kann sichergestellt werden, dass das dem Instrument zugeführte Kältemittel sicher in der gewünschten gasförmigen, flüssigen oder superfluiden Phase vorliegt und unter dem nötigen Druck steht. Ist das Instrument zum Betrieb mit gasförmigem komprimiertem Kältemittel vorgesehen, wird durch die Temperierung des Kältemittels, beispielsweise durch die gezielte Beheizung desselben, ausgeschlossen, dass aus dem Kältemittel flüssige Tropfen auskondensieren, die in Leitungen, in Ventilen oder im Instrument die Funktion behindern könnten. Die Temperiereinrichtung kann dazu als Heizeinrichtung ausgebildet sein. Weiter kann die Temperiereinrichtung dazu vorgesehen werden, in dem Puffergefäß den gewünschten Druck aufzubauen, der höher sein kann als der Druck in dem Vorratsgefäß.

Umgekehrt ist es dank der Temperierung des Kältemittels auch möglich, für Instrumente, die mit flüssigem Kältemittel zu betreiben sind, die Temperatur des Kältemittels so einzuregeln, dass das flüssige Kältemittel in den Leitungen, Ventilen oder im Instrument nicht an unerwünschten Stellen Dampfblasen bildet, die die Funktion beeinträchtigen könnten. Die Temperiereinrichtung kann dazu z.B. als Kühleinrichtung ausgebildet sein.

Das Kältemittel kann einer thermischen Kompression unterzogen werden, indem es mittels der Temperiereinrichtung in dem Puffergefäß unter Volumenabschluss erwärmt wird. Zusätzlich oder alternativ kann es einer mechanischen Kompression unterworfen werden, indem es mittels einer mechanischen Pumpe verdichtet wird.

Zur Bereitstellung des Kältemittels in dem Puffergefäß mit dem gewünschten Druck, d.h. zur mechanischen Kompression, kann zusätzlich zu der Temperiereinrichtung eine Pumpe vorgesehen sein, die saugseitig mit dem Vorratsgefäß und druckseitig mit dem Puffergefäß verbunden ist. Die Pumpe komprimiert dann aus dem Vorratsgefäß entnommenes Kältemittel und führt es dem Puffergefäß mit erhöhtem Druck zu.

In dem Vorratsgefäß kann das Kältemittel, zum Beispiel CO₂ oder ein anderes leicht verflüssigbares Gas, zumindest zu einem Teil in flüssiger Phase und zu einem anderen Teil in gasförmiger Phase vorliegen. Es kann dem Vorratsgefäß in flüssiger oder, wie es meist bevorzugt wird, in gasförmiger Phase entnommen werden. Liegt es in dem Vorratsgefäß sowohl in flüssiger als auch in gasförmiger Phase vor, bildet die gasförmige Phase einen gesättigten Dampf, aus dem bei Druckerhöhung oder Abkühlung Kältemitteltropfen auskondensieren können.

Auch in dem Puffergefäß kann das Kältemittel teilweise in flüssiger und teilweise in gasförmiger Phase oder alternativ auch ausschließlich in gasförmiger Phase oder ausschließlich in flüssiger Phase vorliegen. Das Puffergefäß dient vorwiegend zur Zwischenspeicherung von Kältemittel und zur Temperierung desselben sowie zum Druckausgleich, um von der Pumpe herrührende Druckschwankungen zu vermindern und Kältemittel für die weitere Verwendung bereitzustellen, im Rahmen derer es zum Beispiel zunächst über ein Druckregelventil geführt wird.

Durch Temperierung mindestens des Puffergefäßes vorzugsweise aber auch des Druckregelventils (falls vorhanden) der Pumpe und etwaiger diese Elemente verbindenden Leitungen wird erreicht, dass das Kältemittel von der Flüssig/Gasförmig-Phasengrenze weggeführt wird. Ist das Kältemittel beispielsweise CO₂ und wird ein Anwendungsdruck von z.B. 55 bar benötigt, kann das Kältemittel einer als Vorratsgefäß dienenden Gasflasche nicht in jedem Fall sicher bei dem gewünschten Druck entnommen werden. Beispielsweise liefert eine Gasflasche bei 15°C CO₂ lediglich noch mit einem Druck von 51 bar. Bei dem erfindungsgemäßen Gerät wird das Kältemittel (CO₂) jedoch auch unter solchen Bedingungen mit dem nötigen Druck zuverlässig zur Verfügung gestellt, und zwar durch Druckerhöhung mittels der Pumpe und Temperierung des auf einen höheren Druck verdichteten Kältemittels.

Das Gerät kann z.B. in der zu dem Vorlaufanschluss führenden Leitung einen Dampfsättigungssensor aufweisen, der mit der Steuereinrichtung des Geräts verbunden ist. Der Dampfsättigungssensor erfasst den Zustand des Kältemittels und erzeugt ein entsprechendes Signal. Die Steuereinrichtung kann darauf eingerichtet sein, die Temperiereinrichtung oder etwaige Pumpen und Ventile so zu steuern, dass die gewünschte Dampfsättigung erreicht wird. Das Dampfsättigungsventil kann vor oder nach einem etwaigen Druckregelventil angeordnet sein,

Die Temperierung des Kältemittels in der Pumpe, dem Puffergefäß und/oder dem Druckregler kann eine Beheizung und/oder auch eine Kühlung des Kältemittels beinhalten, insbesondere wenn das Kältemittel für das Instrument in flüssiger Form bereitzustellen ist. In diesem Fall wird das Kältemittel durch Kühlung von der Flüssig/Gasförmig-Phasengrenze weggeführt oder, wenn der Abstand zur Phasengrenze durch Druckerhöhung zu groß wird, durch Erwärmung auch an diese herangeführt.

Die Temperiereinrichtung kann je nach Anwendungsfall eine Heizeinrichtung oder eine Kühleinrichtung oder eine kombinierte Heiz-und-Kühleinrichtung sein. Die Heiz-und-Kühleinrichtung kann durch ein oder mehrere Peltierelemente gebildet sein, die thermisch mit dem mindestens einen Puffergefäß verbunden sind. Wenn es kühlend betrieben wird, bewirkt es das Einströmen von Kältemittel in das Puffergefäß. Das Kältemittel erhöht durch Kühlung seine Dichte und kann, wenn ausreichend gekühlt wird, kondensieren. Wird das Peltierelement danach heizend betrieben und ist das Puffergefäß soweit abgeschlossen, dass kein Kältemittel in das Vorratsgefäß zurückströmen kann, steigt der Druck des Kältemittels.

Insgesamt stellt die Erfindung ein Gerät und ein Verfahren bereit, mit dem der Druck des Kältemittels unabhängig von dem Druck im Vorratsgefäß auf einen gewünschten Wert gebracht werden kann, wobei durch Temperierung des Kältemittels ein gewünschter Abstand zur Flüssig/Gasförmig-Phasengrenze eingestellt werden kann bzw. dieser Abstand in einem gewünschten Bereich gehalten werden kann. Dies gilt sowohl für flüssige als auch für gasförmige Kältemittel, womit sich die Betriebssicherheit von kryochirurgischen Instrumenten auch unter widrigen Bedingungen, beispielsweise bei besonders niedrigen oder auch hohen Umgebungstemperaturen, wesentlich erhöhen lässt. Insbesondere wird die Sicherheit des Betriebs des Instruments unabhängig von der Temperatur eines Kältemittelvorratsgefäßes, beispielsweise einer Gasflasche, ermöglicht.

Einzelheiten von Ausführungsformen der Erfindung ergeben sich aus der nachfolgenden Beschreibung und der zugehörigen Figuren sowie aus Unteransprüchen. Es zeigen:
Figur 1 ein erfindungsgemäßes Gerät zur Speisung eines medizinischen Instruments mit einem Kältemittel, in schematisierter und vereinfachter Darstellung,
Figur 2 bis 4 weitere Ausführungsformen eines Geräts zur Speisung eines medizinischen Instruments, jeweils in schematisierter, vereinfachter Darstellung,
Figur 5 ein schematisiertes Phasendiagramm für ein Kältemittel,
Figur 5a ein Enthalphie-Druck-Diagramm für ein Kältemittel,
Figur 6 die Pumpe des erfindungsgemäßen Geräts
Figur 7 ein erfindungsgemäßes Gerät mit thermischer Kompression des Kältemittels,
Figur 8 eine abgewandelte Ausführungsform eines erfindungsgemäßen Geräts mit thermischer Kompression des Kältemittels,
Figur 9 ein erfindungsgemäßes Gerät mit thermischer Kompression des Kältemittels, in vereinfachter Ausführungsform,
Figur 10 eine weiter abgewandelte Ausführungsform eines erfindungsgemäßen Geräts mit thermischer Kompression des Kältemittels,
Figur 11 einen Dampfsättigungssensor in schematischer Darstellung.

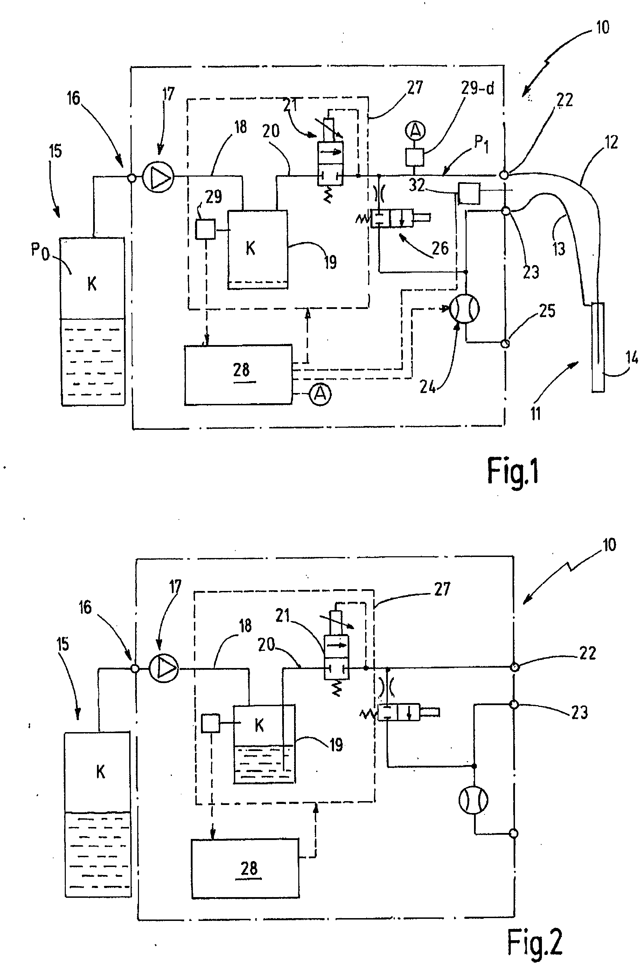

In Figur 1 ist beispielhaft ein Gerät 10 zur Speisung eines kryochirurgischen Instruments 11 veranschaulicht, dem über eine Leitung 12 Kältemittel K zu- und über eine Leitung 13 abgeführt wird. Das Kältemittel K wird dem Instrument 11 dabei unter Druck zugeführt, wobei es in einer Expansionskammer 14 des Instruments 11 expandiert, um Kälte zu erzeugen. Die Kälteerzeugung kann insbesondere auf dem Joule-Thomson-Effekt beruhen, bei dem die Expansion eines gasförmigen Kältemittels K an einer Drossel zur Abkühlung führt.

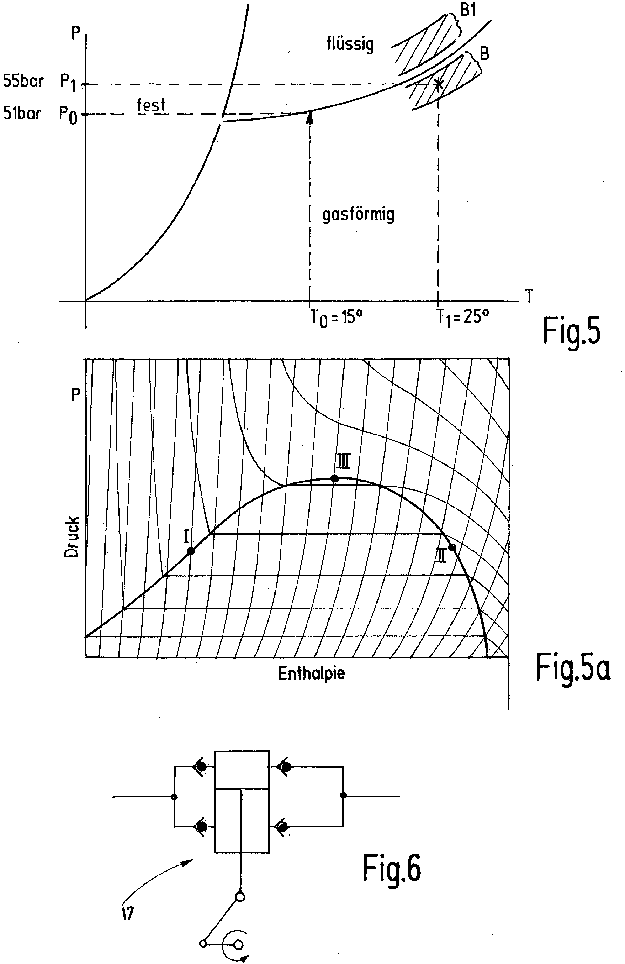

Zur Bereitstellung des Kältemittels K dient ein Vorratsgefäß 15, das zum Beispiel durch eine Gasflasche gebildet ist, die über einen Anschluss 16 mit dem Gerät 10 verbunden ist, um diesem gasförmiges Kältemittel K zuzuführen. Die Gasflasche steht dabei unter einem temperaturabhängigen Druck, der zum Beispiel bei 15°C 51 bar und einem 25°C etwa 64 bar betragen kann. Der Druck P₁ des dem Instrument 11 zuzuführenden Kältemittels K soll beispielsweise 55 bar betragen. Um dies unabhängig vom Druck P₀ in dem Vorratsgefäß 15 sicherzustellen, arbeitet das Gerät 10 mit mechanischer Kompression und weist dazu z.B. eine Pumpe 17 (Druckerhöhungspumpe) auf, die saugseitig über den Anschluss 16 mit dem Vorratsgefäß 15 und druckseitig über eine Leitung 18 an ein Puffergefäß 19 angeschlossen ist. Die Pumpe 17 ist exemplarisch in Figur 6 veranschaulicht. Sie ist als doppelt wirkende Kolbenpumpe ausgebildet, deren Kolben lediglich die Druckdifferenz zwischen dem Vorratsgefäß 15 und dem Puffergefäß 19 aufzubringen hat. Es sind aber auch andere Pumpen, insbesondere Verdrängerpumpen, z.B. Kolbenkompressor, Doppelkolbenkompressor, Schraubenkompressor, Membranpumpe oder auch ein Turbokompressor geeignet.

Von dem Puffergefäß 19 führt eine Leitung 20 vorzugsweise über ein Druckregelventil 21 zu einem Vorlaufanschluss 22, der mit der Leitung 12 verbunden ist. Die den Rücklauf bildende Leitung 13 ist dagegen an einen Rücklaufanschluss 23 gekoppelt, der über einen Strömungsregler (Massendurchflusssensor) 24 an einen Auslass 25 führt. Zur Aktivierung oder Inaktivierung des Instruments 11 dient das Aktivierungsventil 26, das beispielsweise einen Bypass zwischen Vorlauf und Rücklauf öffnet oder schließt.

Das Gerät 10 weist außerdem eine Temperierungseinrichtung 27 auf, die mindestens dem Puffergefäß 19 vorzugsweise aber auch den Leitungen 18, 20 und dem Druckregelventil 21 zugeordnet, d.h. thermisch mit diesen verbunden ist. Die Temperierungseinrichtung 27 ist mit dem Puffergefäß 19, den Leitungen 18, 20 und dem Druckregelventil 21 thermisch verbunden, um deren Temperatur zu beeinflussen. Optional kann die Temperierungseinrichtung 27 auch zusätzlich mit der Pumpe 17 und, falls erforderlich, auch mit der das Druckregelventil 21 mit dem Vorlaufanschluss 22 verbindenden Leitung im thermischen Kontakt stehen. Die Temperierungseinrichtung 27 ist im einfachsten Fall eine Heizeinrichtung, die dazu dient, die mit ihr in thermischem Kontakt stehenden Elemente auf eine gewünschte Temperatur zu bringen und dort zu halten. Sie kann aber auch als Kühleinrichtung oder als kombinierte Heiz- und Kühleinrichtung ausgebildet sein.

Zur Steuerung des Geräts 10 und insbesondere der Temperierungseinrichtung 27 ist eine Steuerungseinrichtung 28 vorgesehen, an die die Temperierungseinrichtung 27 angeschlossen ist. Die Temperierungseinrichtung 27 besteht beispielsweise aus einem oder mehreren Heizelementen, die an den zu beheizenden Teilen und Baugruppen, beispielsweise der Pumpe 17 und/oder der Leitung 18 und/oder dem Puffergefäß 19 und/oder der Leitung 20 und/oder dem Druckregelventil 21 angebracht sind. Die Steuerungseinrichtung 28 kann außerdem mit einem Sensor 29 zur Erfassung des Drucks und/oder der Temperatur des Kühlmittels K in dem Puffergefäß 19 verbunden sein. Ebenso kann die Steuerungseinrichtung 28 mit dem Aktivierungsventil 26 der Pumpe 17 und/oder dem Strömungsregler (Massendurchflusssensor) 24 verbunden sein.

Zur Erläuterung des Betriebs des Geräts 10 und des daran angeschlossenen Instruments 11 wird davon ausgegangen, dass das Instrument 11 mit CO₂ als Kältemittel K bei einem Druck von z.B. 55 bar zu versorgen ist, wobei das Kältemittel im gasförmigen Zustand vorzuliegen hat. Es wird weiter davon ausgegangen, dass die das Vorratsgefäß 15 bildende Gasflasche eine Temperatur von lediglich 15°C aufweist und das Kältemittel K somit bei einem Druck von lediglich 51 bar bereitstellt.

In Betrieb wird das aus dem Vorratsgefäß 15 kommende Kältemittel K nun durch die Pumpe 17 verdichtet, sodass es mit einem Druck von mehr als 55 bar in das Puffergefäß 19 gelangt. In Figur 5 ist dazu das Phasendiagramm von CO₂ veranschaulicht. In dem Vorratsgefäß 15 ist das Kältemittel K sowohl in flüssiger als auch in gasförmiger (dampfförmiger) Phase vorhanden. Sein Zustand liegt somit auf der Phasengrenzlinie zwischen flüssiger Phase und gasförmiger Phase bei T₀ und P₀. Es liegt somit als gesättigter Dampf vor.

Die Verdichtung des Kältemittels in der Pumpe 17 führt zunächst zu einer Temperaturerhöhung, sodass die Druckerhöhung nicht zwangsläufig zur Verflüssigung führen muss. Jedoch kann eine nachfolgende Abkühlung des Kältemittels auf Raumtemperatur eventuell dazu führen, dass es die Phasengrenzlinie überschreitet und Tröpfchen bildet. Durch die Temperierung des Puffergefäßes 19 durch Beheizung desselben wird seine Temperatur jedoch soweit auf die Temperatur T₁ erhöht, beispielsweise auf 25°C, dass es, wie aus Figur 5 hervorgeht, in einem Bereich B des Gebiets für den gasförmigen Aggregatzustand liegt, wobei der Bereich B in einem Sicherheitsabstand zur Phasengrenzlinie angeordnet ist. In dem Puffergefäß 19 ist das Kältemittel K vorzugsweise ausschließlich in gasförmiger Phase vorhanden. Jedoch kann sich an dem Boden des Puffergefäßes 19 auch ein Teil des Kältemittels K zumindest vorrübergehend auch in flüssiger Phase niederschlagen.

Die Steuerungseinrichtung 28 kann darauf eingerichtet sein, die Temperatur und gegebenenfalls auch den Druck in dem Puffergefäß 19 so einzuregulieren, dass der Bereich B (siehe Figur 5) nicht verlassen wird und das Kältemittel K auf seinem Weg bis in die Expansionskammer 14 kein Kondensat bildet. Die Intensität der Kälteerzeugung des Instruments 11 kann dabei über den Strömungsregler (Massendurchflusssensor) 24 reguliert werden, über den der Kältemittelfluss gesteuert wird. Der Strömungsregler 24 kann dabei von der Steuerungseinrichtung 28 gesteuert werden, die ihrerseits beispielsweise mit einem manuell zu bedienenden Aktivierungsschalter verbunden ist.

An dem beschriebenen Gerät 10 und seiner Wirkungsweise sind zahlreiche Abwandlungen möglich. Beispielsweise kann das Gerät 10 dazu ausgelegt sein, dem Vorratsgefäß 15 das Kältemittel K gas- oder dampfförmig zu entnehmen, dem Instrument jedoch an dem Vorlaufanschluss 22 in flüssiger Form bereitzustellen. Dazu kann das Puffergefäß 19 als Kondensationsgefäß ausgebildet sein. In dem Puffergefäß 19 sammelt sich dann Kältemittel K zumindest teilweise in flüssiger Phase. Bei dieser Ausführungsform kann die Temperierungseinrichtung 27 ebenfalls eine Heizung oder auch eine Kühleinrichtung sein. Das Kältemittel kann dem Instrument 11 flüssig zugeführt werden, um in der Expansionskammer 14 zu verdampfen und damit eine entsprechende Kühlwirkung zu erzielen. In diesem Fall vermeidet die Temperierung des Puffergefäßes 19 und gegebenenfalls weiterer Elemente wie der Leitungen 18, 20 und des Druckregulierungsventils 21 eine Dampfblasenbildung in dem Kältemittel K, bevor es in die Expansionskammer 14 gelangt. Die Temperierungseinrichtung 27 stellt sicher, dass der Phasenzustand des Kältemittels K in dem Bereich B1 gemäß Figur 5 liegt. Der Bereich B1 ist in einem Sicherheitsabstand zur Phasengrenzlinie in dem Bereich für den flüssigen Aggregatzustand angeordnet.

In Figur 5a ist ein Enthalphie-Druck-Diagramm eines Kältemittels, beispielsweise CO₂, mit einer Siedelinie I, einer Taulinie II und einem kritischen Punkt III dargestellt. Zwischen der Siedelinie I und der Taulinie II liegt unterhalb des kritischen Punktes III ein Gebiet, in dem das Kältemittel sowohl in flüssiger als auch in dampfförmiger Phase vorliegt. Der Dampfgehalt des Gemischs wird durch die Isovaporen charakterisiert, die die Abszisse schneiden. Die Isothermen durchqueren das Nassdampfgebiet. In diesem lässt sich das Dampf/Flüssigkeits-Verhältnis (d.h. die Dampfsättigung) nicht aus Druck und Temperatur bestimmen. Deswegen lässt sich durch Messung von Druck und Temperatur zumindest in der Nähe der Siedelinie I und der Taulinie II sowie zwischen diesen nicht feststellen, ob das Kältemittel als Gas, als Flüssigkeit oder als Zweiphasengemisch vorliegt. Der korrekte Betrieb des Instruments hängt aber von dem richtigen Zustand des Kältemittels ab. Deswegen kann in der zu dem Vorlaufanschluss 22 führenden Leitung oder an anderer geeigneter Stelle des Geräts 10 ein Dampfsättigungssensor 29-d vorgesehen sein, wie er in Figur 1 angedeutet und an späterer Stelle beschrieben ist. Der Dampfsättigungssensor 29-d kann zusätzlich zu den oder anstelle der anderen Sensoren in dem Gerät 10 vorgesehen sein. Der Sättigungssensor 29-d kann entsprechend an allen Ausführungsformen der erfindungsgemäßen Geräte 10 vorgesehen werden.

Bei der Ausführungsform des Geräts 10 nach Figur 2 muss das Kältemittel K dem Vorratsgefäß 15 nicht zwingend gasförmig entnommen werden. Es ist auch möglich, das Kältemittel K der Pumpe 17 und dem Puffergefäß 19 in flüssiger Form zuzuführen, beispielsweise indem das Vorratsgefäß 15 mit einem entsprechenden Steigrohr versehen oder kopfstehend angeordnet ist.

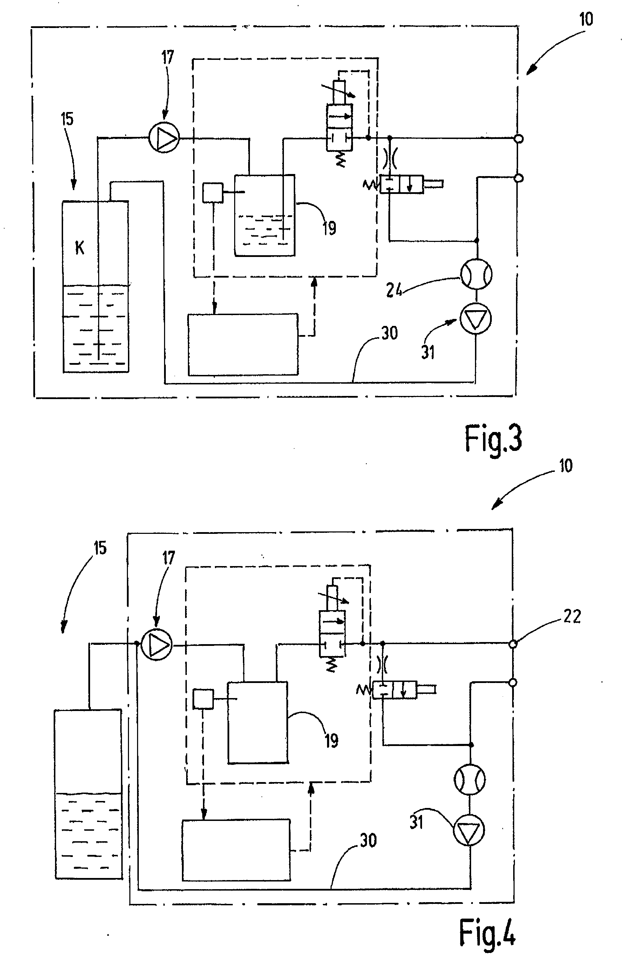

Bei allen beschriebenen Ausführungsformen kann das Vorratsgefäß 15 als gesondertes Gefäß, beispielsweise als Gasflasche, bereitgestellt und an den Anschluss 16 angeschlossen oder alternativ als zu dem Gerät 19 gehöriges Vorratsgefäß ausgebildet sein. Letzteres ist in Figur 3 veranschaulicht, wobei dort kein Auslass vorgesehen ist, um verbrauchtes Kältemittel ins Freie zu entlassen. Vielmehr ist der Strömungsregler/-sensor 24 an eine Rückführungsleitung 30 angeschlossen, in der eine Pumpe 31 angeordnet ist. Die Rückführungsleitung 30 führt zu dem Vorratsgefäß 15, das beispielsweise als Kondensationsgefäß ausgebildet sein kann, um Kältemittel K, wenigstens teilweise, in flüssiger Phase bereitzuhalten. Ansonsten kann das Gerät 10, wie vorstehend zum Beispiel im Zusammenhang mit Figur 2 beschrieben, aufgebaut sein und arbeiten.

Es ist weiter möglich, anstelle des Vorratsgefäßes 15 und des Puffergefäßes 19 sowie der dazwischenliegenden Pumpe 17 lediglich das Puffergefäß 19 vorzusehen und die Rückführungsleitung 30 direkt an das Puffergefäß 19 anzuschließen. In diesem Fall entfallen das Vorratsgefäß 15 und die Pumpe 17, wobei die Pumpe 31 einen zur Speisung des Puffergefäßes 19 ausreichenden Druck aufbaut.

Figur 4 veranschaulicht eine Ausführungsform des Geräts 10 ähnlich wie Figur 3, jedoch mit außen liegendem, nicht zum Gerät gehörigem Vorratsgefäß 15, beispielsweise wiederum in Gestalt einer Gasflasche. Die Rückführungsleitung 30 ist mit dem Sauganschluss der Pumpe 17 verbunden. Alternativ kann sie, wenn die Pumpe 31 einen entsprechenden Druckaufbau gestattet, auch mit dem Puffergefäß 19 verbunden sein. Das Gerät 10 kann, wie Figur 4 veranschaulicht, zur Speisung des Instruments mit gasförmigem Kältemittel K oder auch, nach dem Vorbild der Figur 3, zur Speisung mit flüssigem Kältemittel K eingerichtet sein. Die Temperierungseinrichtung 27 wird von der Steuerungseinrichtung 28 jeweils so gesteuert, dass das Kältemittel in dem Phasendiagramm nach Figur 5 entweder in dem gewünschten sicheren Bereich B oder in dem gewünschten sicher flüssigen Bereich B1 liegt.

Das Gerät 10 kann bei allen Ausführungsformen außerdem eine Instrumentenerkennungseinrichtung 32 aufweisen (Figur 1). Die Instrumentenerkennungseinrichtung 32 kann insbesondere dazu eingerichtet sein, den zum Betrieb des Instruments nötigen Druck zu erfassen oder zu unterscheiden, ob das Instrument 11 mit flüssigem oder mit gasförmigen Kältemittel zu versorgen ist. Dementsprechend kann die Temperiereinrichtung 27 als Heiz-und Kühleinrichtung ausgebildet sein, um das Kältemittel K, je nach Bedarf, in den Bereich B oder B1 des Phasendiagramms nach Figur 5 zu überführen.

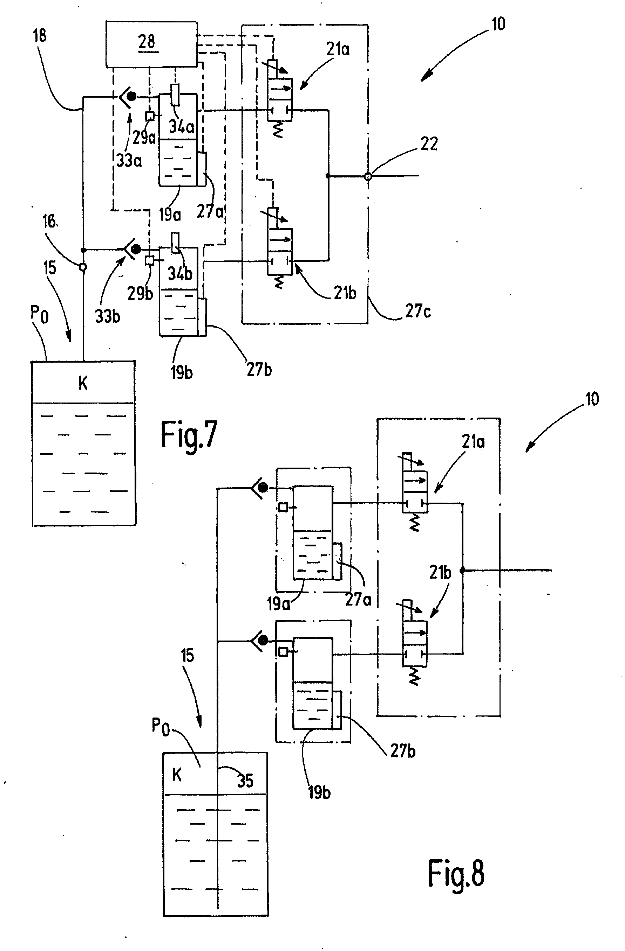

Die bislang beschriebenen Geräte 10 arbeiten mit mechanischer Kompression. In den Figuren 7 und 8 sind hingegen Geräte 10 veranschaulicht, die mit thermischer Kompression des Kältemittels K arbeiten. Dazu weist das Gerät 10 nach Figur 7 ein erstes Puffergefäß 19a sowie ein zweites Puffergefäß 19b auf, die beide jeweils über ein Rückschlagventil 33a bzw. 33b mit dem Vorratsgefäß 15 verbunden sind. Eine mechanische Pumpe in der entsprechenden Leitung 18 ist nicht vorhanden. Bedarfsweise kann jedoch eine solche Pumpe vorgesehen werden. Die Rückschlagventile 33a, 33b sind darauf eingerichtet, eine Strömung aus dem Vorratsgefäß 15 in das jeweilige Puffergefäß 19a, 19b zuzulassen, eine Rückströmung jedoch zu sperren.

Die beiden Puffergefäße 19a, 19b sind jeweils mit einer Temperiereinrichtung 27a, 27b verbunden, die vorzugsweise als Peltier-Element ausgebildet ist. Die beiden dem jeweiligen Puffergefäßen 19a, 19b zugeordnete Temperiereinrichtungen 27a, 27b sind mit der Steuereinrichtung 28 verbunden, um zum Beispiel wechselweise und zeitlich gegeneinander versetzt im Kühlbetrieb oder im Heizbetrieb zu arbeiten. Die als Peltier-Elemente ausgebildeten Temperiereinrichtungen 27a, 27b sind dabei darauf eingerichtet, die Temperatur in dem jeweiligen Puffergefäß 19a, 19b gegenüber der Raumtemperatur zu erhöhen oder abzusenken.

Die beiden Puffergefäße 19a, 19b sind jeweils über Druckregelventile 21a, 21b mit dem Vorlaufanschluss 22 verbunden. Die Druckregelventile 21a, 21b können ebenfalls mit einer gemeinsamen oder separaten Temperiereinrichtung(en) 27c verbunden sein, die vorzugsweise als reine Heizeinrichtung ausgebildet sein kann. Die Druckregelventile 21a, 21b können, wie bei den vorigen Ausführungsformen beschrieben, rein druckgesteuert ausgebildet sein. Zusätzlich oder alternativ können sie von der Steuereinrichtung 28 gesteuert sein, um zu schließen, wenn das jeweilige vorgeschaltete Puffergefäß 19a, 19b nicht beheizt, sondern gekühlt wird. Ansonsten gilt für das Gerät 10 nach Figur 7 die vorige Beschreibung entsprechend.

Das Gerät 10 nach Figur 7 arbeitet wie folgt:

Zur Erläuterung des Betriebs wird zunächst davon ausgegangen, dass beide Puffergefäße 19a, 19b leer sind. Es dringt dann zunächst Kältemittel (CO₂) aus dem Vorratsgefäß 15 über die Leitung 18 und die beiden Rückschlagventile 33a, 33b in die Puffergefäße 19a, 19b vor, bis ein Druckausgleich erreicht ist. Mindestens eines der beiden Puffergefäße 19a, 19b, beispielsweise das Puffergefäß 19a, wird nun mittels der Temperiereinrichtung 27a gekühlt. Das Kältemittel kann dadurch kondensieren, wobei durch den eintretenden Druckabfall weiteres Kältemittel über das Rückschlagventil 33a nachströmt. Es bildet sich in dem Puffergefäß 19a Kondensat, dessen Pegel ansteigt bis der Drucksensor Füllstandssensor 29a anspricht und den Füllstand an die Steuerungseinrichtung 28 meldet. Diese beendet nun die Kühlphase und deaktiviert den Kühlbetrieb der Temperiereinrichtung 27a. Das Gerät 10 ist nun betriebsbereit um ein angeschlossenes Instrument zu speisen. Dazu wird die Temperiereinrichtung 27a in Heizbetrieb gebracht, um das Puffergefäß 19a zu erwärmen und den gewünschten Druck herzustellen. Sogleich kann die Temperiereinrichtung 27b im Kühlbetrieb an Kondensation von zuströmenden Kältemittel K in dem Puffergefäß 19b und somit die Füllung desselben mit Kondensat bewirken bis ein nicht weiter veranschaulichter Füllstandsensor anspricht. Damit kann die Kühlphase an dem Puffergefäß 19b beendet werden.

Der Vorlaufanschluss 22 kann nun unter fortgesetzter Beheizung bzw. Temperierung des Puffergefäß 19a fortgesetzt werden bis das in dem Vorratsgefäß 19 vorhandene Kondensat soweit verbraucht ist, dass der Drucksensor 29 einen zurückgehenden Druck meldet. Die Steuereinrichtung 289 überführt nun die Temperiereinrichtung 27b in Heizbetrieb, um in dem Puffergefäß 19b den nötigen Betriebsdruck aufzubauen. Außerdem aktivieren sie die Temperiereinrichtung 27a im Kühlbetrieb, um das Puffergefäß 19a wieder mit Kältemittelkondensat zu befüllen.

Die Heiz- und Kühlphasen in den beiden Puffergefäßen 19a, 19b können sich somit derart abwechseln, dass an den beiden Druckregelventilen 21a, 21b immer der nötige Eingangsdruck ansteht. Außerdem können die Druckregelventile 21a, 21b mit der Temperiereinrichtung 27c warm gehalten werden, um ein Kondensieren des Kältemittels in den Druckregelventilen 21a, 21b ebenso wie in den zu- und abführenden Leitungen zu verhindern.

Das Gerät 10 nach Figur 8 ist prinzipiell ähnlich aufgebaut. Jedoch weist das Vorratsgefäß 15 Unterschied zu dem Gerät 10 nach Figur 7 eine Steigleitung 35 auf, sodass dem jeweils gekühlten Puffergefäß 19a, 19b flüssiges Kältemittel zugeführt wird. Dies verkürzt die Füllphasen für die Puffergefäße 19a, 19b ebenso wie die für das Füllen des jeweiligen Puffergefäßes 19a, 19b aufzubringende Kühlleistung. Ansonsten gilt die Beschreibung des Geräts 10 nach Figur 7 für das Gerät 10 nach Figur 8 entsprechend.

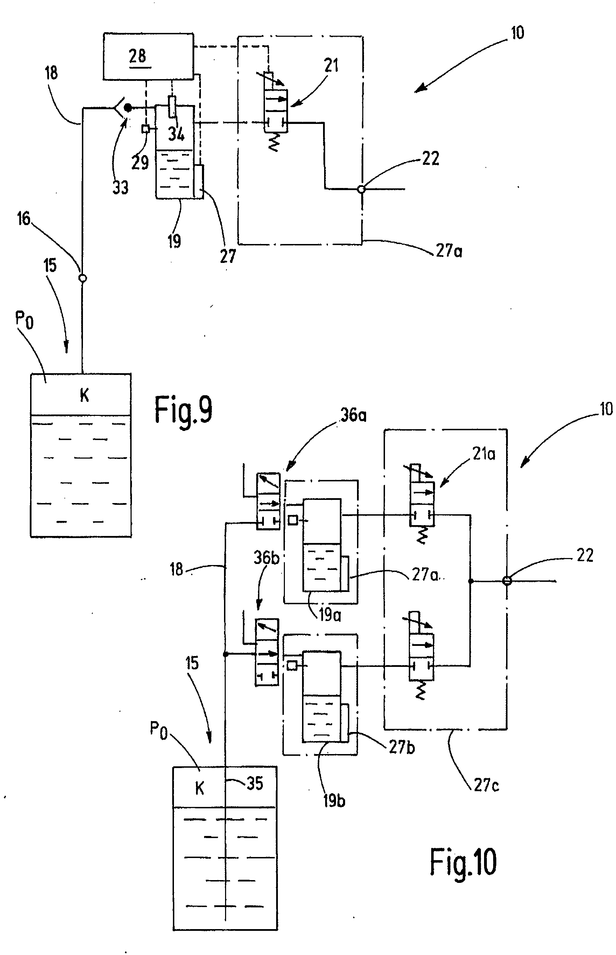

Das Gerät 10 nach Figur 7 ist dazu eingerichtet, an dem Vorlaufanschluss 22 kontinuierlich Gas zu liefern, indem die beiden Puffergefäße 19a, 19b abwechselnd beheizt und gekühlt werden. Ergänzend veranschaulicht Figur 9 eine vereinfachte Version dieses Geräts, das dazu eingerichtet ist, an dem Vorlaufanschluss 22, zumindest für eine gewisse Zeit, Kältemittel mit gewünschtem Druck und Temperatur zu liefern. Zur Temperierung des Kältemittels ist lediglich ein einziger Pufferbehälter 19 vorgesehen, indem durch Kühlung mittels der Temperiereinrichtung 27 ein geringer Druck erzeugbar ist, der Kältemittel in das Puffergefäß 19 einströmen und kondensieren lässt. Alternativ kann das Vorratsgefäß 15, anders als in Figur 9 dargestellt, mit einer Steigleitung 35 versehen oder kopfstehend angeordnet sein, so dass bei Kühlung des Puffergefäßes 19 flüssiges Kältemittel in dieses einströmt.

Nach dieser Füllphase kann das Puffergefäß 19 mittels der Temperiereinrichtung 27 erwärmt werden, so dass sich der gewünschte höhere Druck in dem Puffergefäß 19 einstellt. Es ist nun möglich, an dem Vorlaufanschluss 22 so lange Kältemittel mit gewünschtem Druck und gewünschter Temperatur bereitzustellen bis der Vorrat des Puffergefäßes 19 erschöpft ist. Die Größe des Puffergefäßes kann so groß bemessen sein, dass der vorhandene Vorrat zur Durchführung einer oder mehrerer Operationen ausreicht.

Ein weiteres abgewandeltes Gerät 19 veranschaulicht Figur 10. Die Besonderheit dieses Geräts besteht darin, dass die Puffergefäße 19a, 19b mit Temperiereinrichtungen 27a, 27b verbunden sind, die nur eine Heizfunktion, jedoch keine Kühlfunktion aufweisen, wie beispielsweise elektrischen Heizwiderständen. Anstelle von Rückschlagventilen 33a, 33b sind bei dem Gerät 10 nach Figur 10 jedoch Wegeventile 36a, 36b vorgesehen, die das Puffergefäß 19a, 19b entweder mit dem Vorratsgefäß 15 verbinden oder zur Umgebung hin entlüften. Anstelle der Wegeventile 36a, 36b können auch andere gesteuerte Ventile, beispielsweise jeweils ein Auf/Zu-Ventil in der Leitung 18 und ein anderes gesteuertes Auf/Zu-Ventil zur Entlüftung des jeweiligen Puffergefäßes vorgesehen sein. Die Wegeventile 36a, 36b oder die sonstigen Ventile werden von der in Figur 10 nicht weiter veranschaulichten Steuereinrichtung 28 gesteuert. Zur Erläuterung der Funktionsweise wird zunächst davon ausgegangen, dass beide Puffergefäße 19a, 19b mit Kältemittel gefüllt sind. Wird nun beispielsweise das Puffergefäß 19a mittels der Temperiereinrichtung 27a beheizt und ist das Wegeventil 36a geschlossen, steigt der Druck in dem Puffergefäß 19a auf den gewünschten Wert. Über das Druckregelventil 21a wird nun Kältemittel mit dem gewünschten Druck an den Vorlaufanschluss 22 geliefert.

Zeitgleich kann das Puffergefäß 19b mit Kältemittel gefüllt werden. Dazu wird es zunächst mittels des Wegeventils 36b entlüftet und somit drucklos gemacht. Nachfolgend wird das Puffergefäß 19b über das Wegeventil 36b mit der Leitung 18 verbunden, so dass Kältemittel über die Steigleitung 35 und das Wegeventil 36b in das Puffergefäß 19b einströmen kann bis ein Druckausgleich erfolgt ist. Das Wegeventil 36b kann nun schließen, so dass das Puffergefäß 19b zur Kältemittellieferung einsatzbereit ist. Bevor eine solche gewünscht wird, schaltet die Steuereinrichtung 28 die Heizeinrichtung 27b ein, um das Puffergefäß 19b zu temperieren und somit auf die gewünschte Temperatur und den gewünschten Druck zu bringen, womit das Puffergefäß 19b lieferbereit ist. Es können nun, zumindest kurzzeitig, beide Puffergefäße 19a, 19b Kältemittel liefern, wobei davon ausgegangen wird, dass der Kältemittelvorrat in dem Puffergefäß 19a zur Neige geht oder ganz aufgebraucht ist. Sobald dies der Fall ist, wird die Temperiereinrichtung 27a deaktiviert, so dass das Puffergefäß 19a wieder abkühlen kann. Außerdem kann das Wegeventil 36a nun genutzt werden, um Restdruck aus dem Puffergefäß 19a abzulassen und das Puffergefäß 19 somit drucklos zu machen. Ist dies geschehen, kann das Puffergefäß 19a über das Wegeventil 36a mit dem Vorratsgefäß 15 verbunden werden, so dass Kältemittel in das Puffergefäß 19a nachströmt und dieses so lange füllt bis ein Druckausgleich erreicht ist. Zu diesem Zeitpunkt kann das Wegeventil 36a wieder schließen und somit in die in Figur 10 veranschaulichte Position überführt werden. Nach Aktivierung der Temperiereinrichtung 27a und ausreichender Beheizung des Puffergefäßes 19a durch diese, ist das Puffergefäß 19a lieferbereit. Somit können beide Puffergefäße 19a, 19b wechselweise gefüllt und temperiert werden, um zeitlich alternierend oder auch mit zeitlicher Überschneidung Kältemittel mit gewünschtem Druck und gewünschter Temperatur an den Vorlaufanschluss 22 zu liefern. Bei einer vereinfachten Version des Geräts 10 nach Figur 10 ist lediglich ein einziges Puffergefäß 19 vorhanden, das mittels des Wegeventils 36a abwechselnd drucklos gemacht, dann gefüllt und danach von dem Vorratsgefäß getrennt und beheizt wird, um den aufgenommenen Kältemittelvorrat mit gewünschtem Druck und gewünschter Temperatur bereitzustellen. Die Arbeitsweise ist dann ähnlich wie bei dem Gerät 10 nach Figur 9 mit dem Unterschied, dass die zur Füllung des Puffergefäßes 19a erforderliche Druckabsenkung nicht durch Kühlung des Puffergefäßes 19a, sondern durch Entlüftung zur Atmosphäre hin erreicht wird.

Auch bei dem Gerät 10 nach Figur 9 oder 10 und den davon abgeleiteten abgewandelten Ausführungsformen kann zur Unterstützung der Füllung des jeweiligen Puffergefäßes 19, 19a, 19b in der Leitung 18 eine Pumpe angeordnet werden.

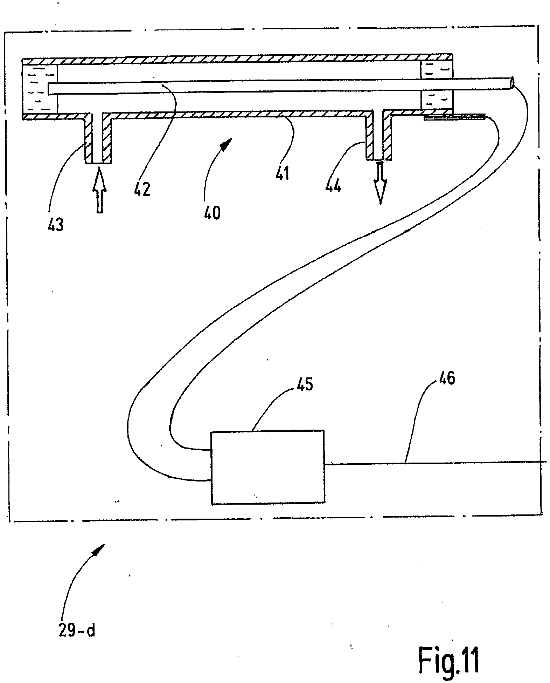

In Figur 11 ist der Dampfsättigungssensor 29-d schematisch veranschaulicht. Der Dampfsättigungssensor 29-d ist als kapazitiver Sensor ausgebildet. Er weist dazu einen Kondensator 40 auf, durch den das Fluid, dessen Zustand zu bestimmen ist, als Dielektrikum durchgeleitet wird. Der Kondensator 40 weist ein rohrförmiges Gehäuse 41 auf, das als äußere genutzt wird und in dessen Innenraum eine zweite zum Beispiel stabförmige Elektrode 42 angeordnet ist. An beiden Enden ist das Gehäuse 41 dicht verschlossen, wobei die Elektrode 42 aus dem Gehäuse 41 herausgeführt ist. Außerdem sind an dem Gehäuse 41 wenigstens zwei Anschlüsse 43, 44 zur Zu- und Abführung des Fluids vorgesehen, dessen Zustand zu bestimmen ist.

Die beiden Elektroden, d.h., das Gehäuse 41 und die Elektrode 42 sind über geeignete elektrische Verbindungen mit einem elektrischen Schaltkreis 45 verbunden, der zur Bestimmung der Kapazität zwischen der Elektrode 42 und dem Gehäuse 41 dient. Der Schaltkreis kann dazu jedes geeignete Kapazitäts-Messverfahren nutzen. In Abhängigkeit von der erfassten Kapazität erzeugt er an seinem Ausgang 46 ein die gemessene Kapazität und somit die Dampfsättigung des durch den Kondensator 40 geleiteten Fluides kennzeichnendes Signal, das der Steuerungseinrichtung 28 zugeleitet wird.

Weiter ist es möglich, einen Temperatursensor vorzusehen, der die Temperatur des den Kondensator 40 durchfließenden Kältemittels in unmittelbarer Nähe des Dampfsättigungssensors 29-d erfasst. Es kann vorgesehen werden, den Druck des Kältemittels wenigstens dann aus der gemessenen Dampfsättigung und der gemessenen Temperatur zu bestimmen, wenn der Zustand des Kältemittels zwischen der Siedelinie I und der Taulinie II liegt (Figur 5a).

Das Gerät 10 zur Speisung kryochirurgischer Instrumente mit einem Kältemittel, insbesondere CO₂, das vorzugsweise in Flaschen bereitgestellt wird, kann eine mechanische oder eine thermische Verdichtereinrichtung aufweisen. Dazu kann eine Pumpe 17 vorgesehen sein, um das der Flasche entnommene CO₂ in einem Puffergefäß 19 mit einem gewünschten Betriebsdruck bereitzustellen, wobei der Druck in der Gasflasche kleiner sein kann als der gewünschte Betriebsdruck. Das Gerät 10 umfasst eine Temperierungseinrichtung 27, die dazu eingerichtet ist, das Kältemittel auf eine gewünschte Temperatur, insbesondere eine Temperatur zu bringen, die höher ist als die Temperatur in der Gasflasche oder einem sonstigen Vorratsgefäß 15. Damit wird eine sichere Versorgung eines kryochirurgischen Instruments mit Kältemittel des gewünschten Drucks und einer ausreichenden Temperatur sichergestellt. Insbesondere werden unerwünschte Tröpfchenbildungen in dem Instrument oder zuführenden Leitungen unterbunden.

### Bezugszeichen:

- 10: Gerät
- 11: Instrument
- 12: Leitung (Vorlauf)
- K: Kältemittel
- 13: Leitung (Rücklauf)
- 14: Expansionskammer
- 15: Vorratsgefäß
- 16: Anschluss
- 17: Pumpe
- 18: Leitung
- 19: Puffergefäß 19a, 19b
- 20: Leitung
- 21: Druckregelventil 21a, 21b
- 22: Vorlaufanschluss
- 23: Rücklaufanschluss
- 24: Strömungsregler/-sensor (Massendurchflusssensor)
- 25: Auslass
- 26: Aktivierungsventil
- 27: Temperierungseinrichtung 27a, 27b, 27c
- 28: Steuerungseinrichtung
- 29: Drucksensor 29a, 29b
- 29-d: Dampfsättigungssensor
- 30: Rückführungsleitung
- 31: Pumpe
- 32: Instrumentenerkennungseinrichtung
- 33a, 33b: Rückschlagventile
- 34a, 34b: Füllstandsensor
- 35: Steigleitung
- 36a, 36b: Wegeventile
- 40: Kondensator
- 41: Gehäuse
- 42: Elektrode
- 43: Anschluss
- 44: Anschluss
- 45: Schaltkreis

## Patentansprüche

1. Gerät (10) zur Speisung eines medizinischen Instruments (11) mit einem Kältemittel (K),
mit einem Vorratsgefäß (15) oder einem Anschluss (16) für ein Vorratsgefäß (15), das zur Speicherung eines ersten Vorrats des Kältemittels K in komprimierter Form mit einem ersten Druck P₀ eingerichtet ist,
mit einem Puffergefäß (19), das zur Speicherung eines zweiten Vorrats des Kältemittels K in komprimierter Form mit einem zweiten Druck P₁ eingerichtet ist,
mit einer Temperierungseinrichtung (27), die wenigstens mit dem Puffergefäß (15) verbunden ist, um dessen Temperatur (T) zu beeinflussen.

2. Gerät nach Anspruch 1, **dadurch gekennzeichnet, dass** eine Pumpe (17) vorgesehen ist, die saugseitig mit dem Vorratsgefäß (15) und druckseitig mit dem Puffergefäß (19) verbunden ist.

3. Gerät nach einem der vorstehenden Ansprüche, **dadurch gekennzeichnet, dass** es einen Dampfsättigungssensor (29-d) aufweist.

4. Gerät nach einem der Ansprüche 1 oder 2, **dadurch gekennzeichnet, dass** der Druck und die Temperatur in dem Puffergefäß derart eingestellt sind, dass das Kältemittel (K) in dem Puffergefäß (19) ausschließlich in gasförmiger Phase vorliegt, oder dass der Druck und die Temperatur in dem Puffergefäß (19) derart eingestellt sind, dass das Kältemittel (K) in dem Puffergefäß (19) zumindest zu einem Teil in flüssiger Phase und zu einem anderen Teil in gasförmiger Phase vorliegt, oder dass der Druck und die Temperatur in dem Puffergefäß (19) derart eingestellt sind, dass das Kältemittel (K) in dem Puffergefäß (19) vollständig in flüssiger Phase vorliegt.

5. Gerät nach Anspruch 2, **dadurch gekennzeichnet, dass** die Pumpe (17) eine Verdrängerpumpe, vorzugsweise eine Doppelkolbenpumpe ist.

6. Gerät nach einem der vorstehenden Ansprüche, **dadurch gekennzeichnet, dass** zwischen dem Puffergefäß (19) und einem Vorlaufanschluss (22) zur Belieferung eines zu speisenden kryochirurgischen Instruments (11) mit Kältemittel K ein Druckregelventil (21) angeordnet ist.

7. Gerät nach Anspruch 6, **dadurch gekennzeichnet, dass** eine Instrumentenerkennungseinrichtung (32) vorgesehen ist, die mit der Pumpe (17) und/oder der Temperierungseinrichtung (27) verbunden ist, um das Kältemittel K in dem für das Instrument (11) vorgesehenen Zustand bereitzustellen.

8. Gerät nach Anspruch 6, **dadurch gekennzeichnet, dass** die Temperierungseinrichtung (27) mit dem Druckregelventil (21) verbunden ist.

9. Gerät nach einem der vorstehenden Ansprüche, **dadurch gekennzeichnet, dass** die Temperierungseinrichtung (27) mit Leitungen (18, 20) verbunden ist, die das Puffergefäß (19) mit der Pumpe (17) und/oder das Puffergefäß (19) mit einem Druckregelventil (21) und oder das Druckregelventil (21) mit einem Vorlaufanschluss (22) verbinden.

10. Gerät nach einem der vorstehenden Ansprüche, **dadurch gekennzeichnet, dass** die Temperierungseinrichtung (27) mindestens eine Heizfunktion aufweisend ausgebildet ist.

11. Gerät nach einem der vorstehenden Ansprüche, **dadurch gekennzeichnet, dass** es einen Rücklaufanschluss (23) zum Anschluss des zu speisenden Instruments (11) aufweist.

12. Gerät nach Anspruch 11, **dadurch gekennzeichnet, dass** der Rücklaufanschluss (23) mit einem Massendurchflusssensor (24) verbunden ist.

13. Gerät nach einem der Ansprüche 11 oder 12, **dadurch gekennzeichnet, dass** zwischen dem Rücklaufanschluss (23) und dem Vorratsgefäß (15) eine Rückspeisepumpe (31) angeordnet ist.

14. Verfahren zur Bereitstellung eines Kältemittels für ein medizinisches Instrument (11), bei welchem Verfahren:
Kältemittel (K) aus einem Vorratsgefäß (15) bei einem ersten Druck P₀ entnommen und in ein Puffergefäß (19) überführt wird,
der Druck des Kältemittels (K) bei der Überführung in das Puffergefäß (19) oder nach der Überführung erhöht wird,
**dadurch gekennzeichnet,**
**dass** das Kältemittel (K) während und/oder nach seiner Verdichtung temperiert wird.

15. Verfahren nach Anspruch 14, **dadurch gekennzeichnet, dass** das Kältemittel als gesättigter Dampf aus dem Vorratsgefäß (15) entnommen und mittels einer Pumpe (17) komprimiert wird, um seinen Druck zu erhöhen.
